# EUROPEAN PATENT APPLICATION

(11) **EP 4 108 250 A1**
(43) Date of publication of application: **28.12.2022**
(21) Application number: 20919437.2
(22) Date of filing: 21.02.2020
(51) Int. Cl.: A61K 36/54, A61P 13/08, A23L 33/105

(54) **COMPOSITION FOR BENIGN PROSTATIC HYPERPLASIA TREATMENT CONTAINING LAURUS NOBILIS EXTRACT AS ACTIVE INGREDIENT, AND FUNCTIONAL HEALTH FOOD**

(71) Applicant: Kosabio Inc., Gyeonggi-do 12106 (KR)
(72) Inventor: KIM, Kyungjae, Namyangju-si Gyeonggi-do 12258 (KR); SONG, Young-Cheon, Seoul 01200 (KR); KONG, Hyun-Seok, Seoul 03730 (KR); KIM, Ji-Yeon, Seoul 02439 (KR); CHOI, Do-Hyun, Pocheon-si, Gyeonggi-do 11173 (KR); AN, Jin-Ho, Seoul 03351 (KR)
(74) Representative: Thun, Clemens
(86) International application number: PCT/KR2020/002567
(87) International publication number: WO 2021/167146

(57) **Abstract**

The present disclosure relates to a composition for benign prostatic hyperplasia treatment containing a Laurus nobilis extract as an active ingredient and to a functional health food.

## Description

### Technical Field

The present disclosure relates to a composition and a health functional food for treatment of benign prostatic hyperplasia, each containing a Laurus nobilis extract as an active ingredient.

### Background Art

Bay laurel (Laurus nobilis) is native to the Mediterranean coast and is mainly distributed in Gyeongnam and Jeonnam regions in the Korean Peninsula. Bay laurel is an evergreen arboreous tree that grows to around 15 m in height and has a dark gray bark and lush branches and leaves. Bay leaves are alternate phyllotaxy, hard, long oval or lanceolate, dark green with wavy edges, and fragrant when rubbed.

Bay leaves have many medicinal uses, but their most impressive benefits include detoxification of the body, protection against bacterial infections, and aiding in fast wound healing. In addition, the leaves are known to have a preventive effect on diabetes mellitus and specific types of cancer.

Bay leaves are used as a diuretic that stimulates urination and reduces body toxicity. Organic compounds found in laurel leaves also relieve irritable bowel syndrome (IBS) and are very effective in alleviating celiac disease (an inherited allergic disease of the small intestine).

One of the greatest benefits of bay leaves is anti-inflammatory efficacy. Bay leaves contain a unique phytonutrient called parthenolide, which can quickly reduce inflammation and irritation when applied topically to infected areas, such as sore joints or those affected by arthritis. In addition, an effect of preventing cancer is also observed. Containing catechins, linalool, parthenolide, and Eugenol, bay leaves can defend the body from free radicals. In other words, the leaves prevent healthy cells from mutating into cancer cells due to free radicals.

In addition, bay leaves are a natural product that has been used for antibacterial, antifungal, and antioxidant performances. Among ingredients found in bay leaves, sesquiterpene lactone inhibits NO production, exhibiting anti-inflammatory activity and enhancing glutathione S-transferase activity in the liver. Costunolide, which is a sesquiterpene lactone, was reported to repress blood ethanol levels as assayed in mice.

The pathogenesis of benign prostatic hyperplasia, although not yet exactly known, is accounted for by enlarged prostate cells induced by excessive production of dihydrotestosterone (DHT). When abundantly present in blood, testosterone is converted by 5α-reductase to a large amount of DHT which, in turn, binds to androgen receptors (AR) in prostate cells to induce benign prostatic hyperplasia. In addition, men decrease in male hormone production with age. Under this condition, the prostate cells of aged men increase the number of ARs in order to maintain the endocrine balance, allowing DHT to bind to more ARs, whereby benign prostatic hyperplasia is caused. As an endogenous factor, the level of an apoptosis regulator in prostate tissues is reportedly increased upon onset of prostate cancer and benign prostatic hyperplasia. Through such pathological mechanisms, the prostate tissue overgrows. Patients with benign prostatic hyperplasia undergo various clinical urination-related symptoms including urinary obstruction, residual urine, detrusor instability, urinary retention, dysuria, and the like and, in severe cases, may suffer from urinary stones, renal failure, hematuria, and infectious complications.

As the aging population increases, prostatic hyperplasia has a significant impact on the quality of life of the male population. Recently, as economic and social conditions have improved, the seriousness of prostatic hyperplasia is recognized as being important, with the consequent expansion of the market for therapeutics therefor.

Until now, there have been a narrow spectrum of therapies for benign prostatic hyperplasia, including alpha-adrenergic receptor blockers that relieve symptoms like dysuria, 5-alpha reductase inhibitors that lower DHT concentrations, and some complementary and alternative therapies.

Examples of alpha adrenergic receptor blockers include terazosin, doxazosin, alfuzosin, and tamsulosin. As 5-alpha reductase inhibitors, finasteride and dutasteride are used. Despite their therapeutic benefits, these drugs have significant restrictions on their use due to their inherent adverse reactions. For instance, side effects include orthostatic hypertension and cardiovascular abnormalities caused by alpha adrenergic receptor blockers and decreased libido caused by 5-alpha reductase inhibitors. For these reasons, complementary and alternative therapeutic agents have been suggested as an alternative, and saw palmetto is used. However, as these therapeutic drugs also have limitations in their efficacy, the need for new therapeutically effective substances is emerging.

Until the present inventors noticed, nothing was known about the effect of laurel on the treatment of benign prostatic hyperplasia. Leading to the present disclosure, intensive and thorough research conducted by the present inventors into development of new therapeutically effective substances resulted in the finding that a bay leaf extract increases immunity and reduces the weight and volume of the prostate, as assayed by cell experiments and benign prostatic hyperplasia-induced animal experiments.

### Disclosure of Invention

### Technical Problem

The present disclosure has been drawn from the background and is to provide a composition that allows a bay leaf extract to be applied to the therapy of benign prostatic hyperplasia as the extract has been proven to be therapeutically effective for benign prostatic hyperplasia.

### Solution to Problem

An aspect of the present disclosure provides a composition containing a bay leaf extract as an active ingredient for treatment and prevention of benign prostatic hyperplasia.

In this regard, the bay leaf extract is an extract obtained by using ethanol as a solvent.

Another aspect of the present disclosure provides a health functional food for palliating benign prostatic hyperplasia.

### Advantageous Effects of Invention

As will be stated below, the bay leaf extract is effective for palliating benign prostatic hyperplasia.

### Brief Description of Drawings

FIGS. 1a and 1b are HPLC chromatograms of 30% and 70% ethanol extracts of bay leaves.
FIG. 2 shows anti-inflammatory effects of 30% and 70% ethanol extracts of bay leaves as assayed for NO production levels of macrophages.
FIGS. 3a and 3b show levels of inflammatory cytokines (IL-1β and IL-6) according to treatment with 30% and 70% ethanol extracts of bay leaves.
FIGS. 4a, 4b, and 4c shows therapeutic effects of the 70% ethanol extract of bay leaves on benign prostatic hyperplasia.
FIGS. 5a, 5b, and 5c shows prophylactic effects of the 70% ethanol extract of bay leaves on benign prostatic hyperplasia.

### Mode for Carrying out the Invention

### <1. Preparation of Bay Leaf Extract >

Bay leaves used in this experiment were purchased from Heungil dang.

Two rounds of extraction from 2.5 kg of bay leaves were conducted, and the extracts thus obtained were pooled. As extraction solvents, ethanol 30% and 70% were used. Bay leaves were added 20-fold volumes of the solvent and heated for 48 hours. The extracts were obtained at yields of 11.8% and 13.0%, respectively. Each of the extracts were filtered, lyophilized, powdered, and stored at -20°C until use. In this experiment, the extracts were dissolved at various concentrations in DMSO (1%) or distilled water (D.W).

### <2. HPLC Analysis of Bay Leaf Extract>

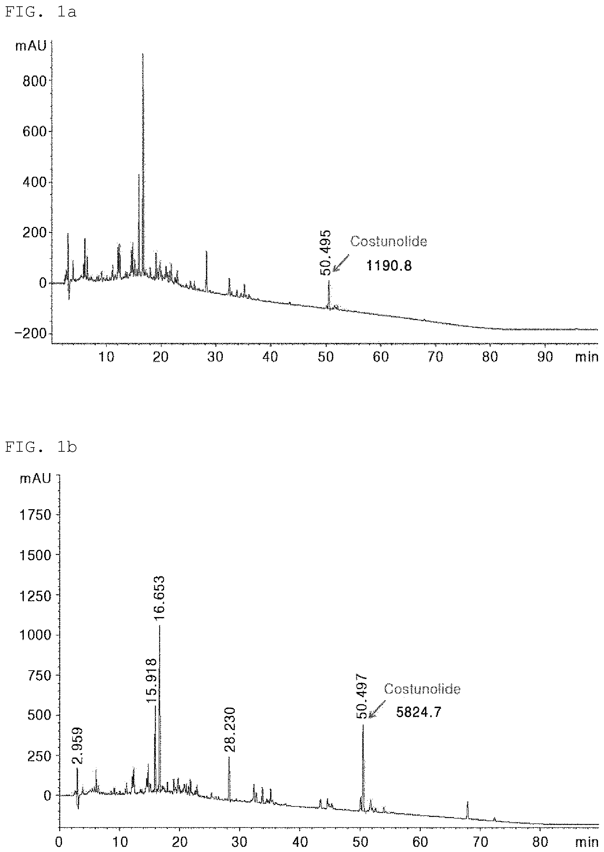

Each of the bay leaf extracts was dissolved at 10 mg/ml and subjected to HPLC to obtain chromatograms (FIG. 1). Each peak in the chromatograms was applied to a calibration curve to calculate concentrations of the ingredients. As a result, the 70% ethanol extract (FIG. 1b) was measured to contain costunolide about 4.9-fold greater than the 30% ethanol extract (FIG. 1a).

### <3. Assay for Anti-Inflammatory Activity of Bay Leaf Extract >

### 1) Culture of macrophage

Macrophage RAW 264.7 cells used in this experiment were purchased from the American Type Culture Collection (ATCC). The cells were cultured in DMEM (high glucose) supplemented with 10% fetal bovine serum (FBS) and 1% penicillin-streptomycin in a CO2 incubator (Formascientific, Inc.). Under a microscope, differentiation of the cells was identified before use in the experiment.

### 2) Assay for anti-inflammatory activity in terms of nitric oxide (NO) level

Activation of the macrophages RAW 264.7 cells was induced by treatment with 200 ng/ml lipopolysaccharide (LPS). The cells were incubated with various concentrations (µg/ml) of the ethanol 30% extract of bay leaves (hereinafter referred to as "EtOH 30%") and the ethanol 70% extract of bay leaves (hereinafter referred to as "EtOH 70%") at 37°C for 18 hours in a 5% CO2 incubator. After 18 hours of incubation, 100 µl of the supernatant from each group was transferred to a new plate. Then, each of the supernatants in new plates was added with 100 µl of a mixture of 1:1 of Greiss reagent A (2% sulfanilamide in 5% phosphoric acid) and Greiss reagent B (0.2% naphthylethylenediamine dihydrochoride), followed by reading absorbance at 540 nm.

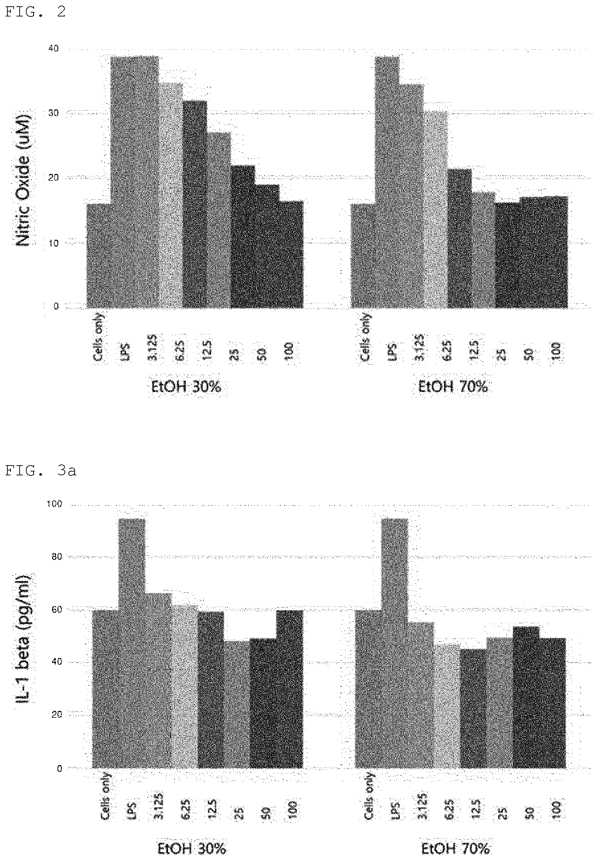

Compared to the control group of non-treated cells, the LPS-treated group was measured to have an increased NO level. The bay leaf extracts both significantly decreased levels of NO in a dosedependent manner (FIG. 2). From the results, it was observed that the bay leaf extracts have anti-inflammatory effects of suppressing excessive inflammatory reactions to prevent tissue injuries.

### 3) Assay for anti-inflammatory activity in terms of inflammatory cytokine level

RAW 264.7 cells were incubated with 200 ng/ml LPS alone and in combination with various concentrations (µg/ml) of the bay leaf extracts (EtOH 30% and EtOH 70%) at 37°C for 18 hours in a 5% CO2 incubator. After 18 hours, the supernatant from each group was measured for levels of inflammatory cytokines IL-1β, IL-6, and TNF-α, using respective ELISA analysis kits (eBioScience 88-7031, 88-7064 USA, BD 555268 USA).

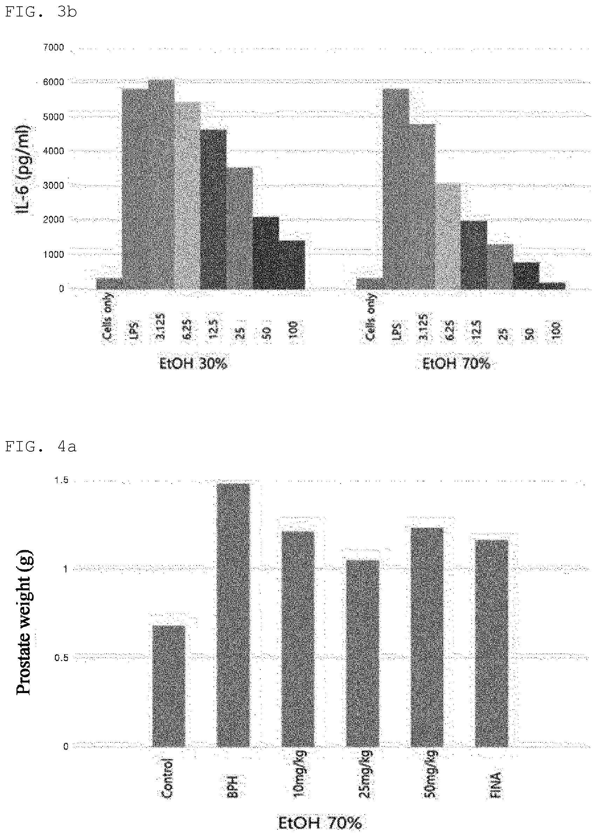

FIG. 3 shows levels of the inflammatory cytokines IL-1β (FIG. 3a) and IL-6 (FIG. 3b) in cultures of LPS-treated macrophages, as measured by ELISA analysis kits. Compared to the cells treated with LPS alone, the cells treated with the bay leaf extracts secreted lower levels of the inflammatory cytokines, demonstrating that the bay leaf extracts have an anti-inflammatory effect.

### <4. Assay for Therapeutic Efficacy of Bay Leaf Extract on Benign Prostatic Hyperplasia>

### 1) Construction of animal model of benign prostatic hyperplasia

Male Wistar rats at 8 weeks of age (Samtako, Korea) were acclimated. When the rats weighed about 380 g on average (19 weeks old), they were divided into groups of 7.

After removing the testis therefrom, the rats in one of the groups were allowed to revive for about 24 hours. Propionate testosterone was subcutaneously injected at a dose of 5 mg/kg into the castrated group and the normal group to induce prostate hypertrophy for 8 weeks (BPH-induced groups). After induction of prostate hypertrophy for 8 weeks, the rats were treated as follows. Along with testosterone injection, the bay leaf extracts were orally administered at predetermined doses (mg/kg) once a day (seven times a week) for 8 weeks. Separately, Finasteride, which is a commercially available therapeutic agent for benign prostatic hyperplasia, was orally administered to a positive control.

### 2) Change in prostate weight and volume

After completion of the experiment, the rats of all the experimental groups were euthanized. The prostate tissues and main organs were excised therefrom and measured for weight and volume using an electronic scale for animal weights and a caliper (mm³) (Table 1) .

As a result, the BPH-induced group was observed to significantly increase in prostate weight and volume, compared to the normal control and thus identified for prostate hypertrophy.

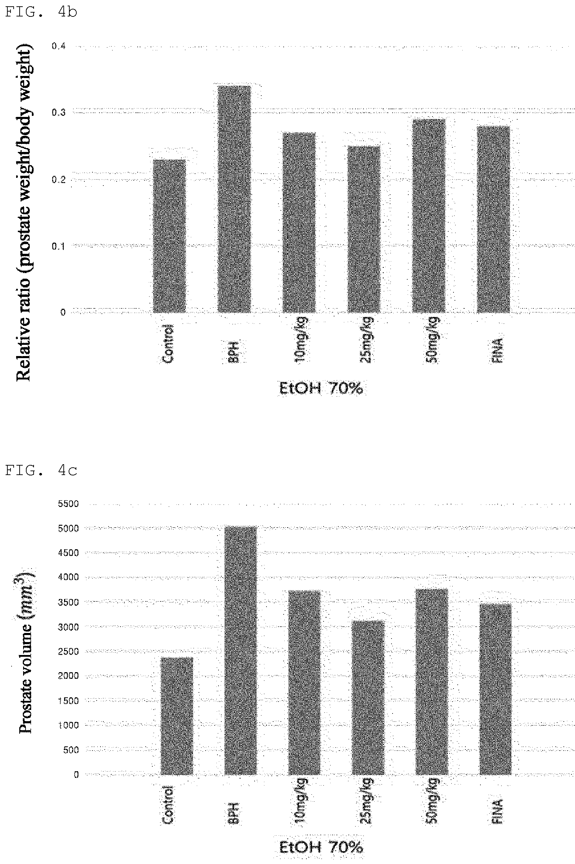

Compared to the BPH-induced group, the bay leaf extract-administered group decreased in prostate weight (FIG. 4a), relative prostate ratio (FIG. 4b), and prostate volume (FIG. 4c), with the similar extent to the BPH drug Finasteride-administered group (FINA). Among others, a dose of 25 mg/kg of the bay leaf extract exhibited higher decreasing effects, compared to the FINA group.

**TABLE 1 (weight: g, ratio: %, volume: mm³)**

| Substance administe red | Test group | Weight ( a) | Prosta te weight (b) | Ratio (b/a*10 0) | Prosta te volume | Live r weig ht | Kidn ey weig ht |
|---|---|---|---|---|---|---|---|
| Non | Contr ol | 291.9 | 0.68 | 0.23 | 2374.3 6 | 8.84 | 0.66 |
| Non | BPH | 438.2 | 1.48 | 0.34 | 5034.1 8 | 10.4 1 | 0.72 |
| EtOH 70% | 10 mg/kg | 444.4 | 1.21 | 0.27 | 3726.0 9 | 10.3 7 | 0.67 |
| | 25 mg/kg | 416.7 | 1.05 | 0.25 | 3124.2 0 | 10.6 7 | 0.71 |
| | 50 mg/kg | 423.5 | 1.23 | 0.29 | 3761 . 28 | 10.3 6 | 0.66 |
| Finasteri de | FINA | 426.6 | 1.16 | 0.28 | 3459.5 9 | 10.3 6 | 0.65 |

### 3) Assay for hepatoxicity and renal function

For use in biochemical index analysis, blood samples were taken from the rats of all the groups through abdominal veins. The blood sample was coagulated for about 30 min and centrifuged at 10,000 rpm for 5 min to separate sera. The sera thus obtained were measured for liver function index (AST and ALT), lipoprotein levels (total cholesterol (T-CHO), HDL cholesterol (HDL-C), LDL cholesterol (LDL-C)), and kidney function index (creatine), using a biochemical analyzer (AU480, Beckman Coulter, USA) (Table 2). As a result, statistically significant changes were observed in none of the extract-administered groups, demonstrating that the extracts are free of hepatotoxicity and nephrotoxicity.

**TABLE 2**

| Substance administere d | Test group | AST (U/L ) | ALT (U/L ) | T-CHO (mg/dL ) | HDL (mg/dL ) | LDL (mg/dL ) | CRE (mg/dL ) |
|---|---|---|---|---|---|---|---|
| Non | Contro 1 | 75.9 | 34.7 | 61.4 | 37.9 | 16.9 | 0.2 |
| Non | BPH | 90.7 | 65.9 | 76.0 | 43.9 | 15.9 | 0.3 |
| EtOH 70% | 10mg/k g | 84.1 | 63.3 | 70.0 | 41.1 | 14.1 | 0.3 |
| | 25mg/k g | 103. 1 | 70.1 | 76.6 | 46.6 | 16.7 | 0.3 |
| | 50mg/k g | 87.3 | 66.7 | 67.6 | 41.3 | 12.3 | 0.3 |
| Finasteride | FINA | 83.1 | 50.3 | 70.7 | 43.6 | 14.1 | 0.3 |

### <5. Assay for Prophylactic Effect of Bay Leaf Extract on Benign Prostatic Hyperplasia>

### 1) Construction of animal model of benign prostatic hyperplasia

Male Wistar rats at 8 weeks of age (Samtako, Korea) were acclimated. When the rats weighed about 320 g on average (12 weeks old), they were divided into groups of 5.

After removing the testis therefrom, the rats in one of the groups were allowed to revive for about 24 hours. Propionate testosterone was subcutaneously injected at a dose of 7 mg/kg into the castrated group and the normal group to induce prostate hypertrophy for 8 weeks (BPH-induced groups). After induction of prostate hypertrophy for 8 weeks, the rats were treated as follows. Along with testosterone injection, the bay leaf extracts were orally administered at predetermined doses (mg/kg) once a day (seven times a week) for 8 weeks. Separately, Finasteride, which is a commercially available therapeutic agent for benign prostatic hyperplasia, was orally administered to a positive control.

### 2) Change in prostate weight and volume

After completion of the experiment, the rats of all the experimental groups were euthanized. The prostate tissues and main organs were excised therefrom and measured for weight and volume using an electronic scale for animal weights and a caliper (mm³) (Table 3).

As a result, the BPH-induced group was observed to significantly increase in prostate weight and volume, compared to the normal control and thus identified for prostate hypertrophy.

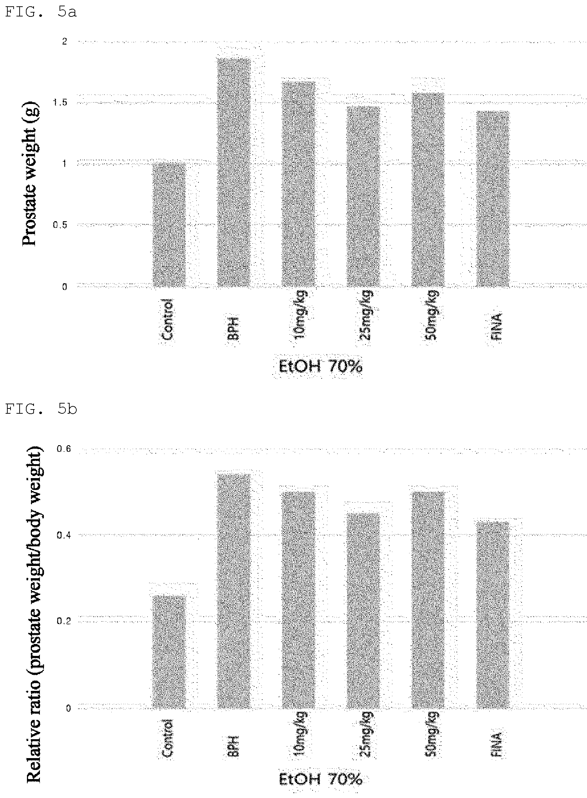

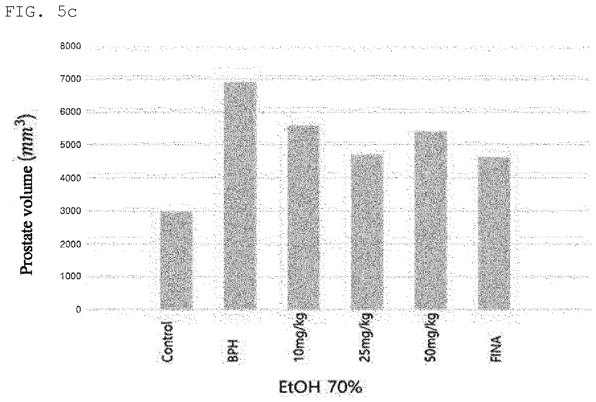

Compared to the BPH-induced group, the bay leaf extract-administered group decreased in prostate weight (FIG. 5a), relative prostate ratio (FIG. 5b), and prostate volume (FIG. 5c). Among others, a dose of 25 mg/kg of the bay leaf extract exhibited higher decreasing effects, compared to the FINA group.

**TABLE 3 (weight: g, ratio: %, volume: mm³)**

| Substance administe red | Test group | Weight ( a) | Prosta te weight (b) | Ratio (b/a*10 0) | Prosta te volume | Live r weig ht | Kidn ey weig ht |
|---|---|---|---|---|---|---|---|
| Non | Contr ol | 393.0 | 1.01 | 0.26 | 2969.3 9 | 8.12 | 0.65 |
| Non | BPH | 343.2 | 1.86 | 0.54 | 6912.6 2 | 7.40 | 0.58 |
| EtOH 70% | 10mg/ kg | 337.3 | 1.67 | 0.50 | 5598.9 4 | 8.05 | 0.68 |
| | 25mg/ kg | 324.8 | 1.47 | 0.45 | 4704.2 1 | 7.08 | 0.61 |
| | 50mg/ kg | 321.7 | 1.58 | 0.50 | 5417.9 7 | 7.23 | 0.59 |
| Finasteri de | FINA | 328.3 | 1.43 | 0.43 | 4626.3 1 | 8.09 | 0.58 |

### 3) Assay for hepatoxicity and renal function

For use in biochemical index analysis, blood samples were taken from the rats of all the groups through abdominal veins. The blood sample was coagulated for about 30 min and centrifuged at 10,000 rpm for 5 min to separate sera. The sera thus obtained were measured for liver function index (AST and ALT), lipoprotein levels (total cholesterol (T-CHO), HDL cholesterol (HDL-C), LDL cholesterol (LDL-C)), and kidney function index (creatine), using a biochemical analyzer (AU480, Beckman Coulter, USA) (Table 4). As a result, statistically significant changes were observed in none of the extract-administered groups, demonstrating that the extracts are free of hepatotoxicity and nephrotoxicity.

**TABLE 4**

| Substance administer ed | Test group | AST (U/L) | ALT (U/L ) | T-CHO (mg/dL ) | HDL (mg/dL ) | LDL (mg/dL ) | CRE (mg/dL ) |
|---|---|---|---|---|---|---|---|
| Non | Contro 1 | 101 . 0 | 40.4 | 75.6 | 54.0 | 15.2 | 0.3 |
| Non | BPH | 118.0 | 47.8 | 67.6 | 48.2 | 18.2 | 0.2 |
| EtOH 70% | 10mg/k g | 112.6 | 48.0 | 71 .2 | 48.8 | 18.4 | 0.2 |
| | 25mg/k g | 138.4 | 46.0 | 65.2 | 46.8 | 18.8 | 0.2 |
| | 50mg/k g | 117.6 | 39.4 | 68.0 | 48.6 | 17.8 | 0.2 |
| Finasterid e | FINA | 140.4 | 51.6 | 60.6 | 43.3 | 17.0 | 0.2 |

Taken together, the data obtained above indicate that the bay leaf extract has a therapeutic and prophylactic effect on benign prostatic hyperplasia as it increases immunity-related, anti-inflammatory reactions and decreases prostate weight and volume.

## Claims

1. A composition comprising a bay leaf extract as an active ingredient for treatment and prevention of benign prostatic hyperplasia.

2. The composition of claim 1, wherein the bay leaf extract is an extract obtained by using ethanol as an extraction solvent.

3. A health functional food comprising the ingredient of claim 1 or 2 for alleviating benign prostatic hyperplasia.
